# EUROPÄISCHE PATENTSCHRIFT

(11) **EP 0 458 176 B1**
(45) Veröffentlichungstag und Bekanntmachung des Hinweises auf die Patenterteilung: **02.08.1995**
(21) Anmeldenummer: 91107829.3
(22) Anmeldetag: 15.05.1991
(51) Int. Cl.: C07C 25/24, C09K 19/30

(54) **Bicyclohexylderivate**
Bicyclohexyl derivatives
Dérivés bicyclohexyliques

(30) Priorität: 21.05.1990 CH 1718/90
(43) Veröffentlichungstag der Anmeldung: 27.11.1991
(73) Patentinhaber: F. HOFFMANN-LA ROCHE AG, 4002 Basel (CH)
(72) Erfinder: Buchecker, Richard, Dr., CH-8008 Zürich (CH); Germann, Alfred, CH-4058 Basel (CH); Schadt, Martin, Dr., CH-4411 Seltisberg (CH); Villiger, Alois, Dr., CH-4055 Basel (CH)
(74) Vertreter: Cottong, Norbert A.

(56) Entgegenhaltungen:
- WO-A-85/04874
- DE-A- 3 139 130
- DE-A- 4 025 550

## Beschreibung

Die vorliegende Erfindung betrifft neue Bicyclohexylderivate, deren Herstellung, flüssigkristalline Gemische, die diese Verbindungen enthalten, sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannung und einen hohen Kontrast ergeben. Weiterhin sollten sie bei üblichen Betriebstemperaturen von etwa -30°C bis etwa +80°C, insbesondere von etwa -20°C bis etwa +60°C eine geeignete Mesophase besitzen, beispielsweise für die oben genannten Zellen eine nematische oder cholesterische Mesophase. Weitere Eigenschaften, wie die elektrische Leitfähigkeit, die dielektrische Anisotropie und die optische Anisotropie, müssen je nach Zellentyp und Anwendungsbereich unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Seit einigen Jahren besteht ein besonderes Interesse an aktiv adressierten Flüssigkristallanzeigen, z.B. TFT-Anwendungen ("thin film transistor" = Dünnfilmtransistor) in Fernsehgeräten. Die Verwendung von Cyano-Verbindungen mit positiver dielektrischer Anisotropie führt jedoch in solchen Anzeigen meist zu einem unerwünscht hohen Stromanstieg. In DE-A-3 139 130 und WO-A-8 504 874 werden flüssigkristalline Halogenbenzolderivate beschrieben, welche sich für obengenannte Zwecke eignen. Ebenso für obige Zwecke geeignete Halogenbenzolderivate werden zudem in EP-A-0 438 563 beschrieben. Dieses Dokument bildet Stand der Technik nach Art. 54(3) EPUe.

Da Flüssigkristalle in der Regel als Mischungen mehrerer Komponenten zur Anwendung gelangen, ist es wichtig, dass die Komponenten untereinander gut mischbar sind.

Gegenstand der Erfindung sind die Verbindungen der allgemeinen Formel 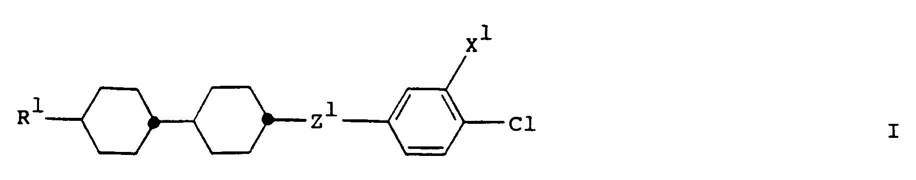
worin Z¹ eine einfache Kovalenzbindung oder -CH₂CH₂- darstellt, X¹ Wasserstoff, Fluor oder Chlor bezeichnet und R¹ 1E-Alkenyl mit 2 bis 12 Kohlenstoffatomen bedeutete, mit Ausnahme von 1-[trans-4-(trans-4-Vinylcyclohexyl)cyclohexyl]-4-chlorbenzol und 1-[trans-4-(trans-4-Vinylcyclohexyl)cyclohexyl]-3-fluor-4-chlorbenzol.

Die erfindungsgemässen Verbindungen sind Flüssigkristalle mit breiten nematischen Phasen und überraschend hohen Klärpunkten. Zugleich besitzen sie überraschend kurze Schaltzeiten insbesondere in Anzeigevorrichtungen mit verdrillt nematischer Struktur. Sie sind niederviskos, gut mischbar mit üblichen Flüssigkristallmaterialien und ermöglichen vergleichsweise niedrige Schwellenspannungen.

Die erfindungsgemässen Verbindungen eignen sich insbesondere als Komponenten nematischer und cholesterischer Gemische und können dank ihrer guten Mischbarkeit untereinander und mit bekannten Flüssigkristallmaterialien in vergleichsweise hohen Konzentrationen verwendet werden.

Der obige Ausdruck "1E-Alkenyl" umfasst geradkettige oder verzweigte Reste. Bevorzugt sind im allgemeinen die geradkettigen Reste, wie Vinyl, 1E-Propenyl, 1E-Butenyl, 1E-Pentenyl, 1E-Hexenyl, 1E-Heptenyl und dergleichen.

Formel I umfasst die Verbindungen der allgemeinen Formeln 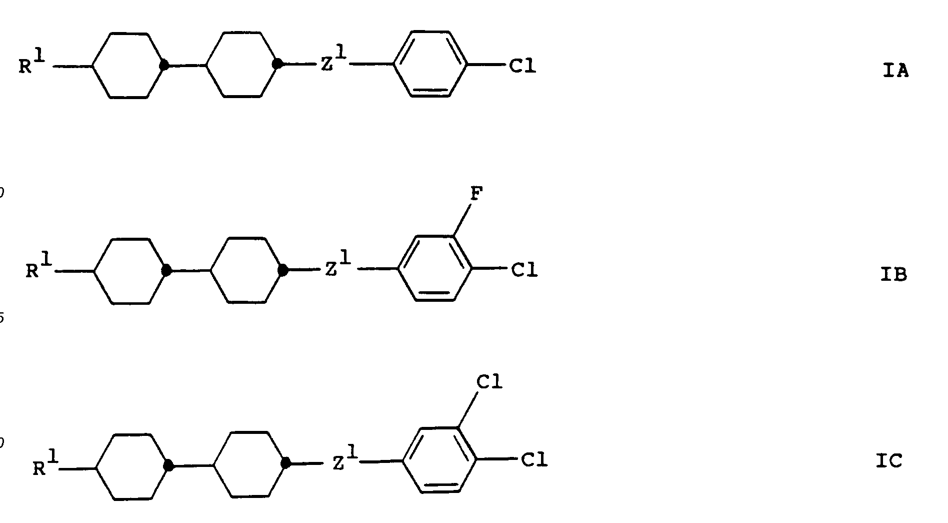
worin R¹ und Z¹ die obigen Bedeutungen haben.

Bevorzugte Reste R¹ in den obigen Formeln sind diejenigen mit 2 bis 7 Kohlenstoffatomen, insbesondere 2 bis 5 Kohlenstoffatomen. Besonders bevorzugt sind Vinyl und 1E-Propenyl.

Die Verbindungen der Formel I können erfindungsgemäss dadurch hergestellt werden, dass man einen Aldehyd der allgemeinen Formel 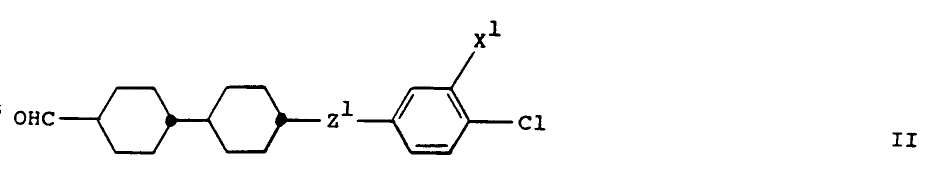
worin X¹ und Z¹ die obigen Bedeutungen haben, in Gegenwart einer Base mit Alkyltriphenylphosphoniumhalogenid umsetzt.

Die Umsetzung mit Alkyltriphenylphosphoniumhalogenid (insbesondere mit dem Alkyltriphenylphosphoniumchlorid, -bromid oder -jodid) in Gegenwart einer Base kann in an sich bekannter Weise erfolgen. Geeignete Basen sind Kalium-tert.-butylat, Natriummethylat, Natriumhydrid, Natriumamid und dergleichen. Die Reaktion wird zweckmässigerweise in einem inerten organischen Lösungsmittel, beispielsweise einem Aether, wie Diäthyläther, Tetrahydrofuran, Dioxan oder tert.Butylmethyläther durchgeführt. Temperatur und Druck sind nicht kritisch, im allgemeinen wird jedoch bei Atmosphärendruck und einer Temperatur von Raumtemperatur bis Rückflusstemperatur gearbeitet.

Wenn R¹ nicht Vinyl bedeutet, werden in der Regel E/Z-Gemische erhalten, welche nach an sich bekannten Methoden, z.B. chromatographisch auf mit Silbernitrat imprägniertem Kieselgel, getrennt werden können. Ferner können gewünschtenfalls die E/Z-Gemische bzw. die Z-Isomere durch Aequilibrierung mit Sulfinsäuren, z.B. Benzolsulfinsäure oder p-Toluolsulfinsäure, überwiegend in die E-Form übergeführt werden.

Die Ausgangsmaterialien der Formel II sind bekannte oder Analoge bekannter Verbindungen und können beispielsweise nach den in den Beispielen beschriebenen Methoden hergestellt werden.

Die Verbindungen der Formel I können in Form von Gemischen untereinander und/oder mit anderen Flüssigkristallkomponenten verwendet werden, wie z.B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Phenylcyclohexane, Cyclohexylcyclohexane, Phenylpyrimidine, Cyclohexylpyrimidine, Phenyldioxane, 2-Cyclohexyl-1-phenyläthane, Terphenyle, Cyclohexylbiphenyle, Cyclohexylphenylpyrimidine und dergleichen. Derartige Substanzen sind dem Fachmann bekannt und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I oder andere Flüssigkristallkomponenten sein. Die Verbindungen der Formel I eignen sich insbesondere für nematische oder, sofern mindestens eine Komponente des Gemisches optisch aktiv ist, auch für cholesterische Gemische.

Aufgrund der guten Löslichkeit der Verbindungen der Formel I und aufgrund ihrer guten Mischbarkeit untereinander kann ihr Anteil in den erfindungsgemässen Gemischen relativ hoch sein. Im allgemeinen ist jedoch ein Anteil von etwa 1-50 Gew.-%, insbesondere etwa 5-30 Gew.-% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formel 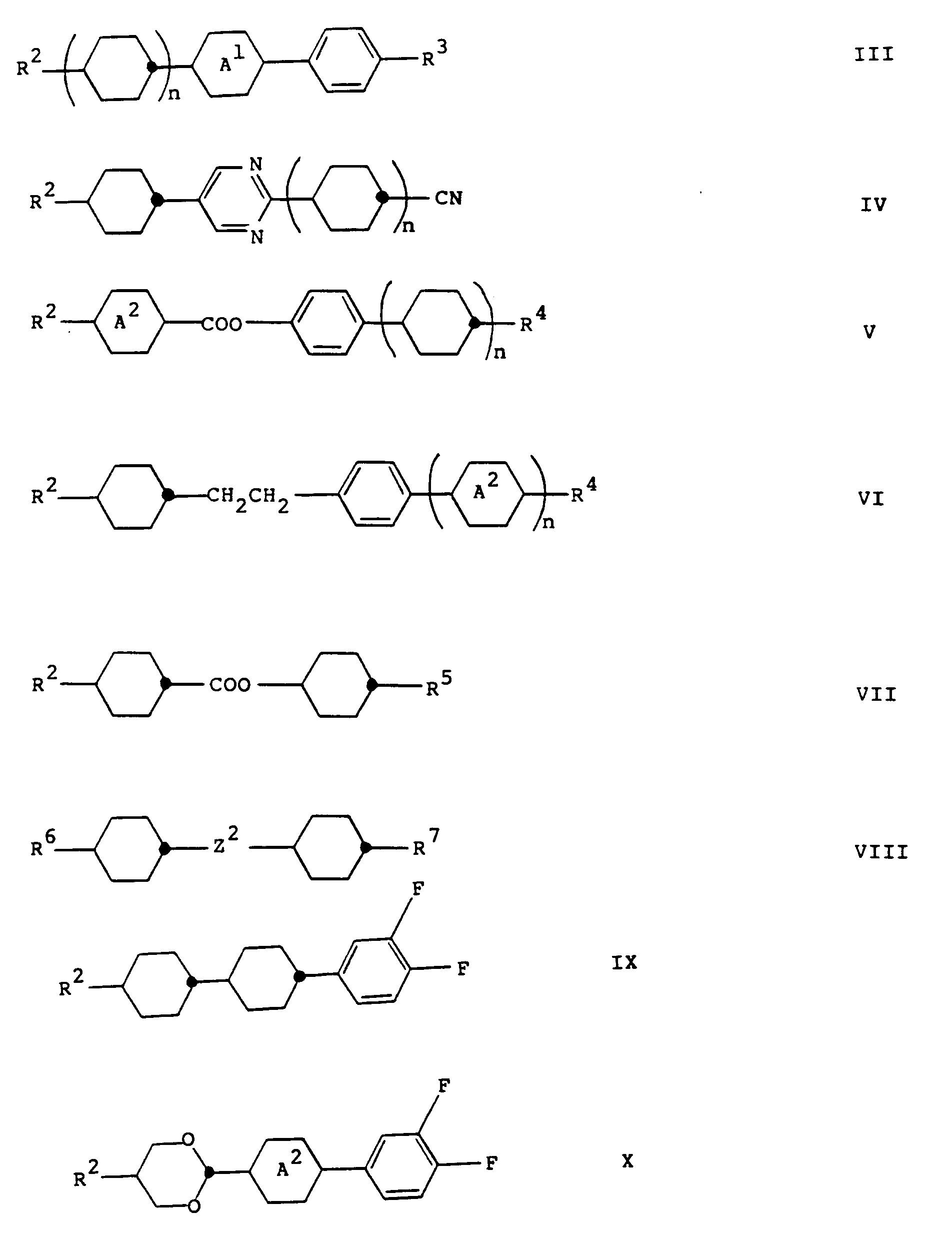 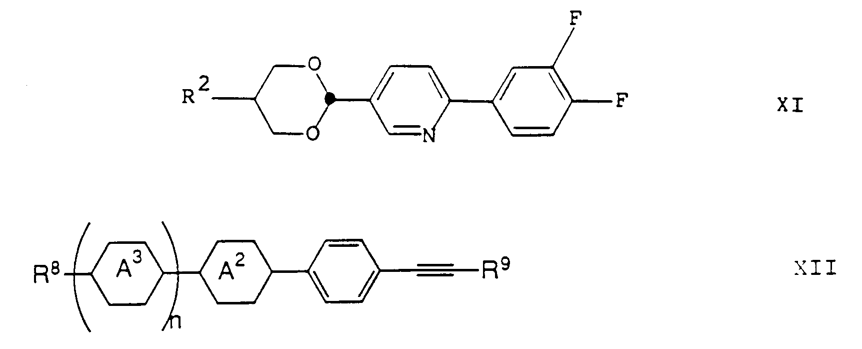
worin n für die Zahl 0 oder 1 steht; R² und R⁵ unabhängig voneinander Alkyl, 3E-Alkenyl, 4-Alkenyl oder an einem gesättigten Ring auch 1E-Alkenyl bedeutet; Ring A¹ 1,4-Phenylen, trans-1,4-Cyclohexylen, Pyrimidin-2,5-diyl oder trans-1,3-Dioxan-2,5-diyl darstellt; R³ Cyano, -NCS, Fluor, Alkyl, Difluormethoxy, Trifluormethoxy, 3E-Alkenyl, 4-Alkenyl, Alkoxy, 2E-Alkenyloxy oder 3-Alkenyloxy bezeichnet; Ring A² 1,4-Phenylen oder trans-1,4-Cyclohexylen darstellt; R⁴ Alkyl, Difluormethoxy, Trifluormethoxy, 3E-Alkenyl, 4-Alkenyl oder an einem Cyclohexanring auch 1E-Alkenyl oder an einem Benzolring auch Cyano, -NCS, Alkoxy, 2E-Alkenyloxy oder 3-Alkenyloxy bedeutet; R⁶ Alkyl, 1E-Alkenyl, 3E-Alkenyl oder 4-Alkenyl bezeichnet; Z² eine einfache Kovalenzbindung oder -CH₂CH₂- darstellt; und R⁷ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl bedeutet; A³ trans-1,4-Cyclohexylen oder trans-1,3-dioxan-2,5-diyl darstellt; R⁸ 3E-Alkenyl, 4-Alkenyl oder, falls n für die Zahl 1 steht, auch 1E-Alkenyl bedeutet; und R⁹ Alkyl bedeutet;
enthält.

Der obige Ausdruck "gesättigter Ring" umfasst trans-1,4-Cyclohexylen und trans-1,3-Dioxan-2,5-diyl. Reste R² bis R⁷ besitzen vorzugsweise höchstens je 12 Kohlenstoffatome, besonders bevorzugt höchstens je 7 Kohlenstoffatome. Geradkettige Reste sind im allgemeinen bevorzugt.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. In den Beispielen bedeutet C eine kristalline, N eine nematische und I die isotrope Phase. V₁₀ bezeichnet die Spannung für 10% Transmission. tₒₙ und t_{off} bezeichnen die Einschaltzeit bzw. die Ausschaltzeit. Δn bezeichnet die optische Anisotropie.

### Beispiel 1

a) 4,53 g Magnesium-Späne und ein Körnchen Jod wurde unter Rühren und Stickstoffbegasung mit 150 ml Tetrahydrofuran versetzt. Anschliessend wurde das Gemisch innert 20 Minuten tropfenweise mit einer Lösung von 38,4 g 4-Brom-1-chlorbenzol in 150 ml Tetrahydrofuran versetzt und 1,25 Stunden am Rückfluss gekocht. Danach wurde das Reaktionsgemisch auf 0°C gekühlt und innert 30 Minuten bei 0-5°C tropfenweise mit einer Lösung 35,7 g 8-(4-Oxocyclohexyl)-1,4-dioxaspiro[4.5]decan in 240 ml Tetrahydrofuran versetzt. Das Reaktionsgemisch wurde noch 4 Stunden ohne Kühlung gerührt, dann innert 10 Minuten mit 240 ml 10-prozentiger Ammoniumchlorid-Lösung versetzt und mit Diäthyläther extrahiert. Die Aether-Phasen wurden zweimal mit Wasser gewaschen, über Natriumsulfat getrocknet, filtriert und eingeengt. Hierbei wurden 54,7 g 1-(4-Chlorphenyl)-4-(1,4-dioxa-8-spiro[4.5]decyl)cyclohexanol als beige Kristalle erhalten.
b) Eine Lösung von 54,7 g 1-(4-Chlorphenyl)-4-(1,4-dioxa-8-spiro[4.5]decyl)cyclohexanol in 570 ml Aethylenchlorid wurde mit 6,95 ml Aethylenglykol und 6,95 g Amberlyst® 15 (stark saures Ionenaustauscher-Harz, Fluka AG) versetzt und 2,3 Stunden durch neutrales Aluminiumoxid rückflussiert. Danach wurde das Reaktionsgemisch auf Raumtemperatur gekühlt, filtriert und dreimal mit Wasser gewaschen. Die wässrigen Phasen wurden zweimal mit Methylenchlorid extrahiert. Die organischen Phasen wurden über Natriumsulfat getrocknet, filtriert und eingeengt. Das erhaltene Rohprodukt (52,9 g) wurde in 150 ml Aethylacetat gelöst. Die Lösung wurde mit Aktivkohle versetzt und warm filtriert. Das Filtrat wurde teilweise eingedampft, dann mit Methanol versetzt und bei Raumtemperatur aufbewahrt. Hierbei wurden 24,9 g 4-Chlor-1-[4-(1,4-dioxa-8-spiro[4.5]decyl) -1-cyclohexenyl]benzol als farbloser kristalliner Niederschlag erhalten. Aufarbeitung der Mutterlauge ergab weitere 6,3 g Produkt als farblose Kristalle.
c) Eine Lösung von 35,0 g 4-Chlor-1-[4-(1,4-dioxa-8-spiro[4.5]decyl) -1-cyclohexenyl]benzol in 1 l Toluol wurde mit 2,6 g Palladium/Kohle (10%) versetzt und bei Raumtemperatur unter Normaldruck bis zum Stillstand der Wasserstoffaufnahme hydriert. Danach wurde das Reaktionsgemisch filtriert und das Filtrat eingeengt, wobei 35,7 g rohes 4-Chlor-1-[4-(1,4-dioxa-8-spiro[4.5]decyl)cyclohexyl]benzol als bräunliches Oel erhalten wurden. Eine Suspension von 32,8 g Aluminiumchlorid in 215 ml Methylenchlorid wurde bei -18°C unter Rühren und Stickstoffbegasung innert 10 Minuten mit einer Lösung des Hydrierungsproduktes (35,7 g bräunliches Oel) in 110 ml Methylenchlorid versetzt und noch 30 Minuten bei 0°C gerührt. Anschliessend wurde das Reaktionsgemisch auf 600 ml Eis/Wasser gegossen, 10 Minuten gerührt und dann dreimal mit Methylenchlorid extrahiert. Die organischen Phasen wurden nacheinander mit Wasser, mit gesättigter Natriumhydrogencarbonat-Lösung und noch zweimal mit Wasser gewaschen, dann über Natriumsulfat getrocknet, filtriert und eingeengt. Hierbei wurden 31,1 g 4-Chlor-1-[trans-4-(1,4-dioxa -8-spiro[4.5]decyl)cyclohexyl]benzol als beige Kristalle erhalten.
d) Ein Gemisch von 31,1 g 4-Chlor-1-[trans-4-(1,4-dioxa -8-spiro[4.5]decyl)cyclohexyl]benzol, 207 ml Toluol und 103 ml Ameisensäure wurde bei Raumtemperatur unter Stickstoffbegasung 1,25 Minuten gerührt. Danach wurde das Reaktionsgemisch auf 500 ml Wasser gegossen und dreimal mit Methylenchlorid extrahiert. Die organischen Phasen wurden einmal mit gesättigter Natriumhydrogencarbonat-Lösung und zweimal mit Wasser gewaschen, über Natriumsulfat getrocknet, filtriert und eingeengt. Hierbei wurden 30,3 g trans-4'-(4-Chlorphenyl)-[1,1'-bicyclohexyl]-4-on als beige Kristalle erhalten.
e) Eine Suspension von 35,5 g Methoxymethyl-triphenylphosphoniumchlorid in 210 ml tert.Butylmethyläther wurde bei -15°C unter Rühren und Stickstoffbegasung mit 12,2 g Kalium-tert.butylat versetzt. Die Suspension wurde noch 30 Minuten bei 5°C gerührt, dann bei 0-5°C innert 40 Minuten tropfenweise mit einer Lösung von 20 g trans-4'-(4-Chlorphenyl)-[1,1'-bicyclohexyl]-4-on in 50 ml Tetrahydrofuran und 200 ml tert.Butylmethyläther versetzt und noch 1 Stunde bei Raumtemperatur gerührt. Anschliessend wurde das Reaktionsgemisch genutscht und das Filtrat eingeengt. Das erhaltene Rohprodukt (45,0 g) wurde mit 250 ml Hexan versetzt. Das Gemisch wurde 10 Minuten bei Raumtemperatur gerührt und genutscht. Einengen des Filtrates ergab 27,0 g Rohprodukt als rot-bräunliches Oel. Chromatographische Reinigung dieses Rohproduktes an Kieselgel mit Hexan und Hexan/Aethylacetat lieferte schliesslich 18,1 g trans-4-(4-Chlorphenyl)-4'-(methoxymethyliden) -[1,1'-bicyclohexyl] als farblose Kristalle.
f) Eine Lösung von 18,1 g trans-4-(4-Chlorphenyl)-4'-(methoxymethyliden) -[1,1'-bicyclohexyl] in 90 ml Tetrahydrofuran wurde mit 22,5 ml 2N Salzsäure versetzt und unter Rühren und Stickstoffbegasung 30 Minuten am Rückfluss gekocht. Anschliessend wurde das Reaktionsgemisch auf Raumtemperatur gekühlt, auf 400 ml Wasser gegossen und dreimal mit Methylenchlorid extrahiert. Die organischen Phasen wurden zweimal mit Wasser gewaschen, über Natriumsulfat getrocknet, filtriert und eingeengt. Der Rückstand (17,0 g) wurde in 150 ml tert.Butylmethyläther gelöst. Die Lösung wurde weitgehend eingedampft und dann mit 200 ml Methanol versetzt. Kristallisation bei -25°C ergab 7,5 g Produkt als farblosen, kristallinen Niederschlag. Aufarbeitung der Mutterlauge lieferte weitere 8,5 g Produkt. Umkristallisation des erhaltenen Produktes ergab schliesslich 13,6 g trans-4'-(4-Chlorphenyl)-[1,1'-bicyclohexyl] -trans-4-carboxaldehyd als farblose Kristalle.
g) Eine Suspension von 2,0 g Aethyltriphenylphosphoniumbromid in 275 ml tert.Butylmethyläther wurde unter Rühren und Stickstoffbegasung mit 6,75 g Kalium-tert.butylat versetzt und noch 1 Stunde bei Raumtemperatur gerührt. Anschliessend wurde die Suspension bei 2°C innert 45 Minuten tropfenweise mit einer Lösung von 10,0 g trans-4'-(4-Chlorphenyl)-[1,1'-bicyclohexyl] -trans-4-carboxaldehyd in 40 ml Tetrahydrofuran und 100 ml tert.Butylmethyläther versetzt und noch 45 Minuten unter Kühlung mit einem Eisbad gerührt. Danach wurde das Reaktionsgemisch genutscht und das Filtrat im Vakuum eingeengt. Der beige, kristalline Rückstand (21,7 g) wurde mit 250 ml Hexan versetzt. Das Gemisch wurde 10 Minuten gerührt und dann genutscht. Einengen des Filtrates im Vakuum ergab 11,6 g Rohprodukt als beige Kristalle. Chromatographische Reinigung an Kieselgel mit Hexan lieferte 9,5 g 1-[trans-4-(trans-4-(1-Propenyl)cyclohexyl)cyclohexyl] -4-chlorbenzol mit einem E/Z-Verhältnis von 11:88 als farblose Kristalle.
h) Eine Lösung von 9,5 g 1-[trans-4-(trans-4-(-1-Propenyl)cyclohexyl)cyclohexyl] -4-chlorbenzol in 115 ml Toluol wurde bei Raumtemperatur unter Rühren und Stickstoffbegasung mit 9,4 ml 2N Salzsäure und 1,43 g Benzolsulfinsäure-Natriumsalz versetzt und 2,25 Stunden erwärmt (Oelbadtemperatur 60-65°C). Danach wurde das Reaktionsgemisch auf Raumtemperatur gekühlt, auf 150 ml 10-prozentige Natriumhydrogencarbonat-Lösung gegossen und dreimal mit Diäthyläther extrahiert. Die organischen Phasen wurden zweimal mit Wasser gewaschen, über Natriumsulfat getrocknet, filtriert und eingeengt. Umkristallisation des erhaltenen Rohproduktes (9,7 g) aus tert.Butylmethyläther/Isopropanol ergab 7,6 g 1-[trans-4-(trans-4-(1E-Propenyl)cyclohexyl)cyclohexyl] -4-chlorbenzol als farblose Kristalle; Smp. (C-N) 106,9°C, Klp. (N-I) 228,5°C.

In analoger Weise können folgende Verbindungen hergestellt werden:
1-[trans-4-(trans-4-(1E-Pentenyl)cyclohexyl)cyclohexyl]-4-chlorbenzol, Smp. (C-N) 89,4°C, Klp. (N-I) 207,1°C;
1-[trans-4-(trans-4-(1E-Propenyl)cyclohexyl)cyclohexyl]-3-fluor-4-chlorbenzol, Smp. (C-N) 90,8°C, Klp. (N-I) 195°C;
1-[trans-4-(trans-4-(1E-Pentenyl)cyclohexyl)cyclohexyl]-3-fluor-4-chlorbenzol, Smp. (C-N) 64,8°C, Klp. (N-I) 173,5°C;
1-[trans-4(trans-4-Vinylcyclohexyl)cyclohexyl]-3,4-dichlorbenzol, Smp. (C-N) 64,3°C; Klp. (N-I) 79°C;
1-[trans-4-(trans-4-(1E-Propenyl)cyclohexyl)cyclohexyl]-3,4-dichlorbenzol, Smp. (C-N) 76,3°C; Klp. (N-I) 126,5°C;
1-[trans-4-(trans-4-(1E-Pentenyl)cyclohexyl)cyclohexyl]-3,4-dichlorbenzol, Smp. (C-N) 52,6°C, Klp. (N-I) 113,4°C.

### Beispiel 2

a) Eine Suspension von 133,3 g Aluminiumchlorid in 250 ml Methylenchlorid wurde bei 20°C unter Kühlung und Stickstoffbegasung tropfenweise mit einer Lösung von 174,2 g 4-Phenylcyclohexanon in 250 ml Methylenchlorid versetzt. Das Reaktionsgemisch wurde noch 30 Minuten gerührt, bis eine klare Lösung erhalten wurde. Inzwischen wurde eine Suspension von 133,3 g Aluminiumchlorid in 500 ml Methylenchlorid unter Stickstoffbegasung mit 172 ml Oxalylchlorid versetzt. Dieses Gemisch wurde anschliessend bei 20-25°C innert 1 Stunde tropfenweise mit der obigen Lösung des 4-Phenylcyclohexanon-Aluminiumchlorid-Komplexes versetzt. Das Reaktionsgemisch wurde noch 30 Minuten gerührt, dann auf 2°C gekühlt und innert 20 Minuten unter Rühren tropfenweise zu einer Lösung von 300 g Kalziumchlorid in 1 l Wasser zugesetzt. Das Gemisch wurde noch 1,5 Stunden bei Raumtemperatur gerührt und dann auf 500 ml Eis/Wasser gegossen. Die organische Phase wurde abgetrennt und mit 500 ml halbgesättigter Kochsalz-Lösung gewaschen. Die wässrigen Phasen wurden zweimal mit je 500 ml Methylenchlorid nachextrahiert. Die vereinigte organische Phase wurde über Natriumsulfat getrocknet, filtriert und eingeengt. Das erhaltene ölige 4-(4-Oxocyclohexyl)benzoylchlorid wurde in 400 ml Toluol gelöst und diese Lösung zu einem Gemisch von 150 ml Methanol, 200 ml Pyridin und 1 l Toluol zugetropft. Das Reaktionsgemisch wurde über Nacht stehengelassen und dann nacheinander mit Wasser, 3N Salzsäure, halbgesättigter Natriumhydrogencarbonat-Lösung und nochmals mit Wasser gewaschen, über Natriumsulfat getrocknet, filtriert und eingeengt. Umkristallisation des Rückstandes aus Toluol bei -25°C ergab 128 g reinen 4-(4-Oxocyclohexyl)benzoesäure-methylester mit Smp. 91-92°C.
b) Eine Suspension von 236 g trockenem Methoxymethyl-triphenylphosphoniumchlorid in 900 ml Tetrahydrofuran wurde unter Stickstoffbegasung mit 77 g Kalium-tert.butylat versetzt. Die Suspension wurde noch 30 Minuten gerührt, dann auf 0°C gekühlt und innert 30 Minuten tropfenweise mit einer Lösung von 128 g 4-(4-Oxocyclohexyl)benzoesäure-methylester in 420 ml Tetrahydrofuran versetzt. Das Reaktionsgemisch wurde noch 3 Stunden bei 0°C gerührt, dann mit 550 ml gesättigter Natriumhydrogencarbonat-Lösung versetzt und dreimal mit Diäthyläther extrahiert. Die organischen Phasen wurden mit Wasser gewaschen, über Natriumsulfat getrocknet, filtriert und eingeengt. Der Rückstand wurde in 2,5 l Hexan und 0,8 l Methanol/Wasser (Vol. 4:1) gelöst. Die Hexan-Phase wurde mit 300 ml Methanol/Wasser (Vol. 4:1) gewaschen, über Natriumsulfat getrocknet, filtriert und eingeengt. Hierbei wurden 123 g 4-[4-(Methoxymethyliden)cyclohexyl]benzoesäure-methylester mit Smp. 58°C erhalten.
c) Ein Gemisch von 123 g 4-[4-(Methoxymethyliden)cyclohexyl]benzoesäure-methylester, 1 l Toluol und 500 ml Ameisensäure wurde über Nacht bei Raumtemperatur kräftig gerührt. Die Ameisensäure-Phase wurde abgetrennt. Die Toluol-Phase wurde mit Wasser neutral gewaschen, über Natriumsulfat getrocknet, filtriert und eingeengt. Das erhaltene, rohe cis/trans-Gemisch wurde in 600 ml Methanol gelöst. Die Lösung wurde unter Stickstoffbegasung bei 3°C innert 10 Minuten tropfenweise zu 1,2 l einer 0,1N methanolischen Kaliumhydroxid-Lösung zugesetzt. Das Reaktionsgemisch wurde noch 1 Stunde bei 3°C gerührt, dann auf Eis/Wasser gegossen und mit Diäthyläther extrahiert. Die organischen Phasen wurden mit Wasser neutral gewaschen, über Natriumsulfat getrocknet, filtriert und eingeengt. Umkristallisation des Rückstandes aus tert.Butylmethyläther ergab 88,5 g 4-(trans-4-Formylcyclohexyl)benzoesäure-methylester mit Smp. 84-85°C.
d) Eine Lösung von 88,5 g 4-(trans-4-Formylcyclohexyl)benzoesäure-methylester in 700 ml Toluol und 25 ml Diäthylenglykol wurde unter Rühren mit 1,5 ml 10-prozentiger (v/v) Schwefelsäure versetzt und 1 Stunde zum Sieden erhitzt, wobei feuchtes Toluol abdestilliert und durch Zusatz von frischem Toluol ersetzt wurde. Anschliessend wurde die Reaktionslösung mit 1 ml Triäthylamin versetzt. Das Gemisch wurde dreimal mit je 150 ml Wasser gewaschen, über Natriumsulfat getrocknet, filtriert und eingeengt. Zweimalige Umkristallisation des Rückstandes aus tert.Butylmethyläther bei -25°C gab 75,4 g reinen 4-[trans-4-(1,3-Dioxolan-2-yl)cyclohexyl]benzoesäure -methylester mit Smp. 110-111°C.
e) Ein Gemisch von 100 g 4-[trans-4-(1,3-Dioxolan-2-yl)cyclohexyl]benzoesäure -methylester, 10 g 5% Rhodium/Aluminiumoxid, 1 l Methanol und 1 ml Triäthylamin wurde bei 60°C unter 10 bar Wasserstoff hydriert, bis die Wasserstoffaufnahme zum Stillstand kam. Das Gemisch wurde filtriert und das Filtrat eingeengt. Hierbei wurden 97 g trans-4-(1,3-Dioxolan-2-yl)-[1,1'-bicyclohexyl] -4-carbonsäure-methylester (cis/trans-Verhältnis 60:40) erhalten, welche mit 19,3 g Methanol und 3,4 ml 10-prozentiger Natriumhydroxid-Lösung versetzt wurden. Unter Rühren und Stickstoffbegasung wurde das Lösungsmittel abdestilliert, bis ein Siedepunkt von 90°C erreicht war. Anschliessend wurde das Gemisch noch 1,5 Stunden bei dieser Temperatur gerührt. Das erhaltene, halbfeste Gemisch wurde langsam abgekühlt und dabei vorsichtig mit 300 ml Methylenchlorid versetzt. Anschliessend wurde das Gemisch mit 150 ml gesättigter Natriumhydrogencarbonat-Lösung gewaschen, über Natriumsulfat getrocknet, filtriert und eingeengt. Umkristallisation des Rückstandes aus 800 ml Hexan ergab 80,7 g reinen 4'-(1,3-Dioxolan-2-yl)-[1,1'-bicyclohexyl] -trans-4-carbonsäure-methylester mit Smp. 126-127°C.
f) Eine Suspension von 3,4 g Lithiumaluminiumhydrid in 200 ml absolutem Diäthyläther wurde innert 2 Stunden tropfenweise mit einer Lösung von 26,7 g trans-4'-(1,3-Dioxolan-2-yl)-[1,1'-bicyclohexyl] -trans-4-carbonsäure-methylester in 500 ml absolutem Diäthyläther versetzt. Das Reaktionsgemisch wurde noch 2 Stunden gerührt und dann vorsichtig auf 25 ml Eis/Wasser und 60 ml 25-prozentige Salzsäure gegossen. Die wässrige Phase wurde abgetrennt und mit Diäthyläther extrahiert. Die vereinigten organischen Phasen wurden mit Wasser neutral gewaschen, über Natriumsulfat getrocknet, filtriert und eingeengt. Hierbei wurden 23,7 g trans-4'-(1,3-Dioxolan-2-yl)-[1,1'-bicyclohexyl] -trans-4-carbinol erhalten.
g) Eine Suspension von 34,5 g Pyridiniumchlorochromat in 150 ml Methylenchlorid wurde innert 30 Minuten tropfenweise mit einer Lösung von 23,7 g trans-4'-(1,3-Dioxolan-2-yl)-[1,1'-bicyclohexyl] -trans-4-carbinol versetzt. Das Reaktionsgemisch wurde noch 3 Stunden bei Raumtemperatur gerührt, dann auf 500 ml absoluten Diäthyläther gegossen und vom Rückstand abdekantiert. Der Rückstand wurde noch dreimal mit je 100 ml Diäthyläther gewaschen. Filtration und Einengen der Lösung ergab 19,8 g trans-4'-(1,3-Dioxolan-2-yl)-[1,1'-bicyclohexyl] -trans-4-carboxaldehyd.
h) Eine Lösung von 29,4 g Triphenylphosphin in 200 ml Toluol wird tropfenweise mit 20,5 g 4-Chlorbenzylbromid versetzt. Das Gemisch wird 3 Stunden auf 65°C und dann 2 Stunden zum Sieden erhitzt. Nach dem Abkühlen auf Raumtemperatur wird der Niederschlag abfiltriert, mit Toluol gewaschen und am Vakuum getrocknet. Das erhaltene 4-Chlorbenzyl-triphenylphosphoniumbromid wird in 500 ml Diäthyläther suspendiert. Die Suspension wird unter Stickstoffbegasung mit 9,5 g Kalium-tert.butylat versetzt, noch 30 Minuten bei Raumtemperatur gerührt und dann bei 2°C tropfenweise mit einer Lösung von 19,8 g trans-4'-(1,3-Dioxolan-2-yl)-[1,1'-bicyclohexyl] -trans-4-carboxaldehyd in 250 ml absolutem Diäthyläther versetzt. Das Reaktionsgemisch wird noch 2,5 Stunden bei Raumtemperatur gerührt. Anschliessend wird das Gemisch mit 500 ml halbgesättigter Natriumhydrogencarbonat-Lösung und mit 300 ml Wasser gewaschen, über Natriumsulfat getrocknet, filtriert und eingeengt. Der Rückstand wird in 200 ml Hexan und 120 ml Methanol/Wasser (Vol. 4:1) gelöst. Die Hexan-Phase wird abgetrennt, mit 40 ml Methanol/Wasser (Vol. 4:1) gewaschen, über Natriumsulfat getrocknet, filtriert und eingeengt. Chromatographische Reinigung des Rückstandes an Kieselgel mit Hexan/Aethylacetat (Vol. 9:1) ergibt trans-4-(1,3-Dioxolan-2-yl)-trans-4'-(4-chlorstyryl) -[1,1'-bicyclohexyl].
i) Eine Lösung von 23,6 g trans-4-(1,3-Dioxolan-2-yl)-trans-4'-(4-chlorstyryl) -[1,1'-bicyclohexyl] in 1,2 l Dioxan und 2,5 ml Triäthylamin wird bei Raumtemperatur und unter Normaldruck mit 2,5 g 5% Palladium/Kohle hydriert, bis die Wasserstoffaufnahme zum Stillstand kommt. Filtration des Reaktionsgemisches und Einengen des Filtrates ergibt trans-4-(1,3-Dioxolan-2-yl)-trans-4'-(4-chlorphenäthyl) -[1,1'-bicyclohexyl].
j) Ein Gemisch von 12,3 g trans-4-(1,3-Dioxolan-2-yl)-trans-4'-(4-chlorphenäthyl) -[1,1'-bicyclohexyl], 300 ml Toluol und 50 ml Ameisensäure wird unter Stickstoffatmosphäre über Nacht gerührt. Anschliessend wird die Ameisensäure-Phase abgetrennt. Die Toluol-Phase wird mit Wasser und mit gesättiger Natriumhydrogencarbonat-Lösung gewaschen, über Natriumsulfat getrocknet, filtriert und eingeengt. Der erhaltene trans-4'-(4-Chlorphenäthyl)-[1,1'-bicyclohexyl] -trans-4-carboxaldehyd wird aus Methanol umkristallisiert.
k) In analoger Weise zu Beispiel 1g) und 1h) wird trans-4'-(4-Chlorphenäthyl)-[1,1'-bicyclohexyl] -trans-4-carboxaldehyd zu 1-[2-[trans-4-(trans -4-(1E-Propenyl)cyclohexyl)cyclohexyl]äthyl]-4-chlorbenzol, Smp. (C-N) 81,4°C, Klp. (N-I) 186°C umgesetzt.

In analoger Weise können folgende Verbindungen hergestellt werden:
1-[2-[trans-4-(trans-4-Vinylcyclohexyl)cyclohexyl]äthyl]-4-chlorbenzol, Smp. (C-N) 62,6°C, Klp. (N-I) 147°C;
1-[2-[trans-4-(trans-4-Vinylcyclohexyl)cyclohexyl]äthyl]-3-fluor-4-chlorbenzol, Smp. (C-N) 49,4°C, Klp. (N-I) 127,5°C;
1-[2-[trans-4-(trans-4-(1E-Propenyl)cyclohexyl)cyclohexyl]äthyl]-3-fluor-4-chlorbenzol, Smp. (C-N) 72°C, Klp. (N-I) 165°C;
1-[2-[trans-4-(trans-4-Vinylcyclohexyl)cyclohexyl]äthyl]-3,4-dichlorbenzol;
1-[2-[trans-4-(trans-4-(1E-Propenyl)cyclohexyl)cyclohexyl]äthyl]-3,4-dichlorbenzol.

### Beispiel 3

Zur Untersuchung der Eigenschaften der Verbindungen der Formel I in Gemischen wurden binäre Mischungen (BM) mit 4-(trans-4-Pentylcyclohexyl)benzonitril hergestellt. Die Schwellenspannung und die Ansprechzeiten wurden in einer TN-Zelle (low bias tilt) mit 8 »m Plattenabstand bei 22°C gemessen; als Betriebsspannung wurde der 2,5-fache Wert der Schwellenspannung gewählt. Die entsprechenden Daten für reines 4-(trans-4-Pentylcyclohexyl)benzonitril betragen:
Klp. (N-I) 54,6°C, V₁₀ = 1,62 V, tₒₙ = 30 ms, t_{off} = 42 ms, Δn = 0,120.

### BM-1

- 90 Gew.-%: 4-(trans-4-Pentylcyclohexyl)benzonitril,
- 10 Gew.-%: 1-[trans-4-(trans-4-Vinylcyclohexyl)cyclohexyl]-4-chlorbenzol;

Klp. (N-I) 63,5°C, V₁₀ = 1,65 V, tₒₙ = 26 ms, t_{off} = 40 ms, Δn = 0,125.

### BM-2

- 80 Gew.-%: 4-(trans-4-Pentylcyclohexyl)benzonitril,
- 20 Gew.-%: 1-[trans-4-(trans-4-Vinylcyclohexyl)cyclohexyl]-4-chlorbenzol;

Klp. (N-I) 73,8°C, V₁₀ = 1,69 V, tₒₙ = 24 ms, t_{off} = 39 ms, Δn = 0,126.

### BM-3

- 90 Gew.-%: 4-(trans-4-Pentylcyclohexyl)benzonitril,
- 10 Gew.-%: 1-[trans-4-(trans-4-(1E-Propenyl)cyclohexyl)cyclohexyl]-4-chlorbenzol;

Klp. (N-I) 66,1°C, V₁₀ = 1,66 V, tₒₙ = 27 ms, t_{off} = 36 ms, Δn = 0,127.

### BM-4

- 80 Gew.-%: 4-(trans-4-Pentylcyclohexyl)benzonitril,
- 20 Gew.-%: 1-[trans-4-(trans-4-(1E-Propenyl)cyclohexyl)cyclohexyl]-4-chlorbenzol;

Klp. (N-I) 78,4°C, V₁₀ = 1,84 V, tₒₙ = 21 ms, t_{off} = 36 ms, Δn = 0,123.

### BM-5

- 90 Gew.-%: 4-(trans-4-Pentylcyclohexyl)benzonitril,
- 10 Gew.-%: 1-[trans-4-(trans-4-(1E-Pentenyl)cyclohexyl)cyclohexyl]-4-chlorbenzol;

Klp. (N-I) 64,5°C, V₁₀ = 1,64 V, tₒₙ = 29 ms, t_{off} = 40 ms, Δn = 0,124.

### BM-6

- 80 Gew.-%: 4-(trans-4-Pentylcyclohexyl)benzonitril,
- 20 Gew.-%: 1-[trans-4-(trans-4-(1E-Pentenyl)cyclohexyl)cyclohexyl]-4-chlorbenzol;

Klp. (N-I) 75,3°C, V₁₀ = 1,89 V, tₒₙ = 24 ms, t_{off} = 40 ms, Δn = 0,125.

### BM-7

- 90 Gew.-%: 4-(trans-4-Pentylcyclohexyl)benzonitril,
- 10 Gew.-%: 1-[trans-4(trans-4-Vinylcyclohexyl)cyclohexyl]-3-fluor-4-chlorbenzol;

Klp. (N-I) 59,9°C, V₁₀ = 1,58 V, tₒₙ = 23 ms, t_{off} = 40 ms, Δn = 0,123.

### BM-8

- 80 Gew.-%: 4-(trans-4-Pentylcyclohexyl)benzonitril,
- 20 Gew.-%: 1-[trans-4-(trans-4-Vinylcyclohexyl)cyclohexyl]-3-fluor-4-chlorbenzol;

Klp. (N-I) 66,3°C, V₁₀ = 1,62 V, tₒₙ = 25 ms, t_{off} = 40 ms, Δn = 0,125.

### BM-9

- 90 Gew.-%: 4-(trans-4-Pentylcyclohexyl)benzonitril,
- 10 Gew.-%: 1-[trans-4(trans-4-(1E-Propenyl)cyclohexyl)cyclohexyl]-3-fluor-4-chlorbenzol;

Klp. (N-I) 62,7°C, V₁₀ = 1,61 V, tₒₙ = 24 ms, t_{off} = 39 ms, Δn = 0,125.

### BM-10

- 90 Gew.-%: 4-(trans-4-Pentylcyclohexyl)benzonitril,
- 10 Gew.-%: 1-[trans-4-(trans-4-(1E-Pentenyl)cyclohexyl)cyclohexyl]-3-fluor-4-chlorbenzol;

Klp. (N-I) 62,0°C, V₁₀ = 1,60 V, tₒₙ = 23 ms, t_{off} = 39 ms, Δn = 0,123.

### BM-11

- 90 Gew.-%: 4-(trans-4-Pentylcyclohexyl)benzonitril,
- 10 Gew.-%: 1-[trans-4-(trans-4-Vinylcyclohexyl)cyclohexyl]-3,4-dichlorbenzol;

Klp. (N-I) 55,2°C, V₁₀ = 1,49 V, tₒₙ = 27 ms, t_{off} = 44 ms, Δn = 0,121.

### BM-12

- 90 Gew.-%: 4-(trans-4-Pentylcyclohexyl)benzonitril,
- 10 Gew.-%: 1-[trans-4-(1E-Propenyl)cyclohexyl)cyclohexyl]-3,4-dichlorbenzol;

Klp. (N-I) 58,5°C, V₁₀ = 1,55 V, tₒₙ = 27 ms, t_{off} = 47 ms, Δn = 0,122.

### BM-13

- 90 Gew.-%: 4-(trans-4-Pentylcyclohexyl)benzonitril,
- 10 Gew.-%: 1-[2-[trans-4-(trans-4-Vinylcyclohexyl)cyclohexyl]äthyl]-4-chlorbenzol,

Klp. (N-I) 61°C, V₁₀ = 1,75 V, tₒₙ = 23 ms, t_{off} = 39 ms, Δn = 0,124.

### BM-14

- 80 Gew.-%: 4-(trans-4-Pentylcyclohexyl)benzonitril,
- 20 Gew.-%: 1-[2-[trans-4-(trans-4-Vinylcyclohexyl)cyclohexyl]äthyl]-4-chlorbenzol,

Klp. (N-I) 69,9°C, V₁₀ = 1,89 V, tₒₙ = 23 ms, t_{off} = 36 ms, Δn = 0,125.

### BM-15

- 90 Gew.-%: 4-(trans-4-Pentylcyclohexyl)benzonitril,
- 10 Gew.-%: 1-[2-[trans-4-(trans-4-(1E-Propenyl)cyclohexyl)cyclohexyl)äthyl]-4-chlorbenzol,

Klp. (N-I) 64,2°C, V₁₀ = 1,74 V, tₒₙ = 25 ms, t_{off} = 40 ms, Δn = 0,126.

### BM-16

- 80 Gew.-%: 4-(trans-4-Pentylcyclohexyl)benzonitril,
- 20 Gew.-%: 1-[2-[trans-4-(trans-4-(1E-Propenyl)cyclohexyl)cyclohexyl]äthyl]-4-chlorbenzol,

Klp. (N-I) 71,1°C, V₁₀ = 1,93 V, tₒₙ = 23 ms, t_{off} = 37 ms, Δn = 0,128.

## Patentansprüche

1. Verbindungen der allgemeinen Formel 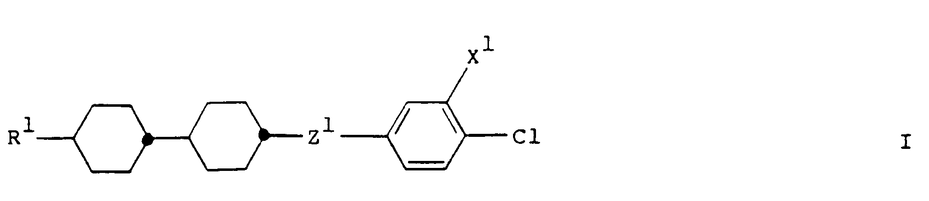 worin Z¹ eine einfache Kovalenzbindung oder -CH₂CH₂- darstellt, X¹ Wasserstoff, Fluor oder Chlor bezeichnet und R¹ 1E-Alkenyl mit 2 bis 12 Kohlenstoffatomen bedeutet, mit Ausnahme von 1-[trans-4-(trans-4-Vinylcyclohexyl)cyclohexyl]-4-chlorbenzol und 1-[trans-4-(trans-4-Vinylcyclohexyl)cyclohexyl]-3-fluor-4-chlorbenzol.

2. Verbindungen nach Anspruch 1, dadurch gekennzeichnet, dass R¹ einen geradkettigen Rest bedeutet.

3. Verbindungen nach Anspruch 1 oder 2, dadurch gekennzeichnet, dass R¹ 2-7, vorzugsweise 2-5 Kohlenstoffatome aufweist.

4. Flüssigkristallines Gemisch mit mindestens 2 Komponenten, dadurch gekennzeichnet, dass mindestens eine Komponente eine Verbindung der in Anspruch 1 definierten Formel I ist.

5. Flüssigkristallines Gemisch nach Anspruch 4, dadurch gekennzeichnet, dass der Anteil an Verbindungen der Formel I 1-50 Gew.-%, vorzugsweise 5-30 Gew.-% beträgt.

6. Flüssigkristallines Gemisch nach Anspruch 4 oder 5, dadurch gekennzeichnet, dass es eine oder mehrere Verbindungen der Formel I und eine oder mehrere Verbindungen aus der Gruppe der Verbindungen der allgemeinen Formeln 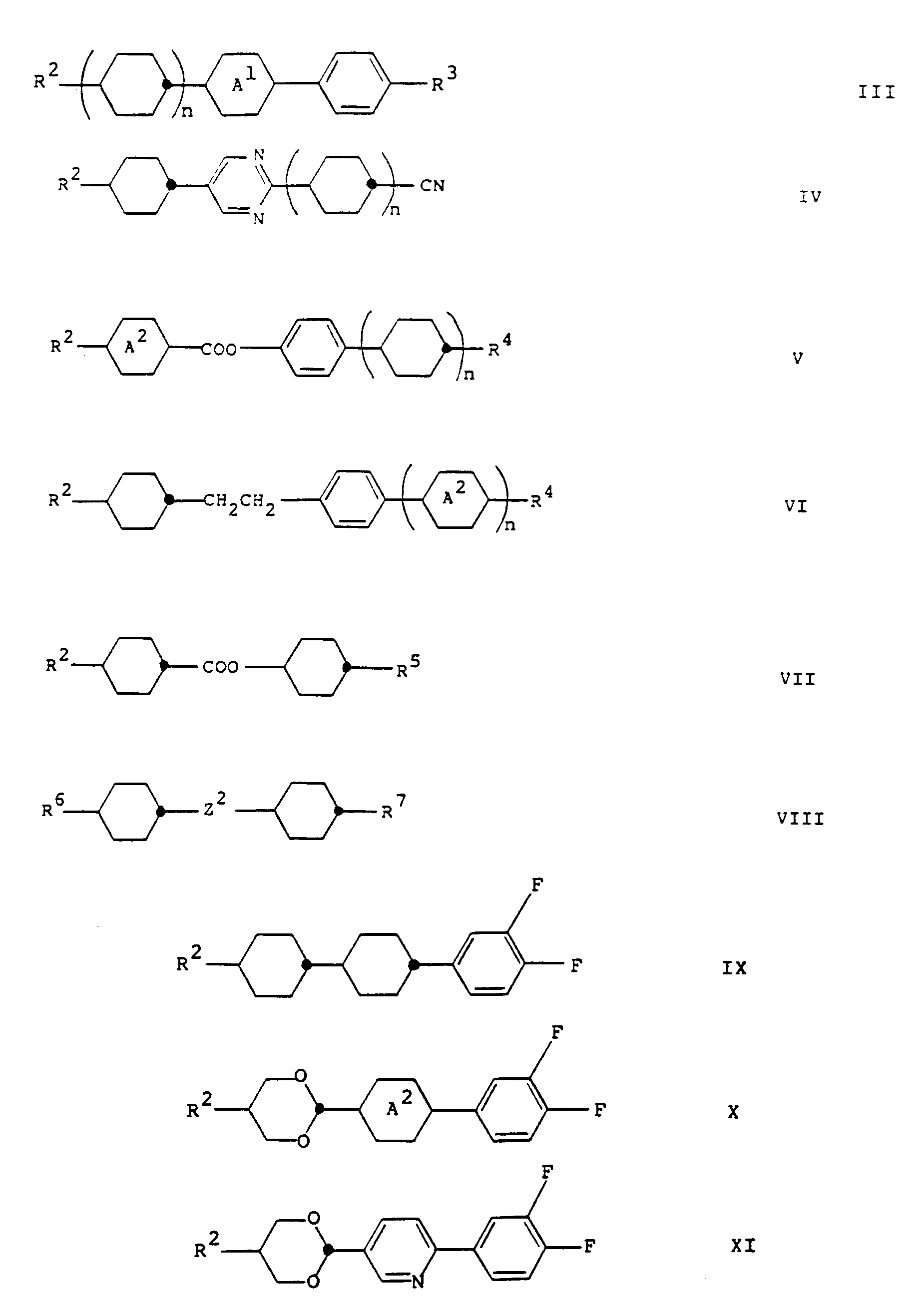 worin n für die Zahl 0 oder 1 steht; R² und R⁵ unabhängig voneinander Alkyl, 3E-Alkenyl, 4-Alkenyl oder an einem gesättigten Ring auch 1E-Alkenyl bedeutet; Ring A¹ 1,4-Phenylen, trans-1,4-Cyclohexylen, Pyrimidin-2,5-diyl oder trans-1,3-Dioxan-2,5-diyl darstellt; R³ Cyano, -NCS, Fluor, Alkyl, 3E-Alkenyl, 4-Alkenyl, Alkoxy, 2E-Alkenyloxy oder 3-Alkenyloxy bezeichnet; Ring A² 1,4-Phenylen oder trans-1,4-Cyclohexylen darstellt; R⁴ Alkyl, 3E-Alkenyl, 4-Alkenyl oder an einem Cyclohexanring auch 1E-Alkenyl oder an einem Benzolring auch Cyano, -NCS, Alkoxy, 2E-Alkenyloxy oder 3-Alkenyloxy bedeutet; R⁶ Alkyl, 1E-Alkenyl, 3E-Alkenyl oder 4-Alkenyl bezeichnet; Z² eine einfache Kovalenzbindung oder -CH₂CH₂- darstellt; und R⁷ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl bedeutet;
enthält.

7. Flüssigkristallines Gemisch nach Anspruch 4 oder 5, dadurch gekennzeichnet, dass es eine oder mehrere Verbindungen der Formel I und eine oder mehrere Verbindungen aus der Gruppe der Verbindungen der allgemeinen Formeln 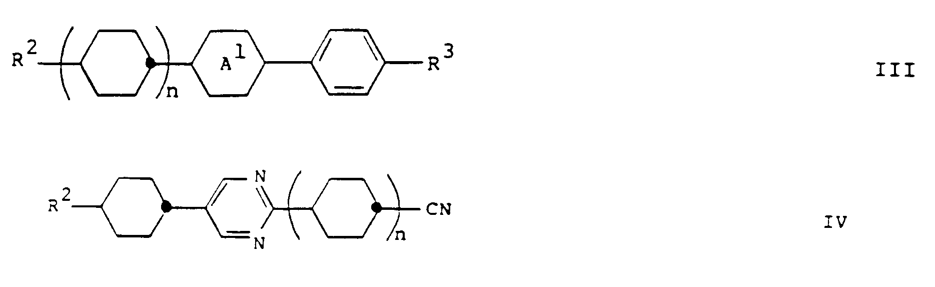 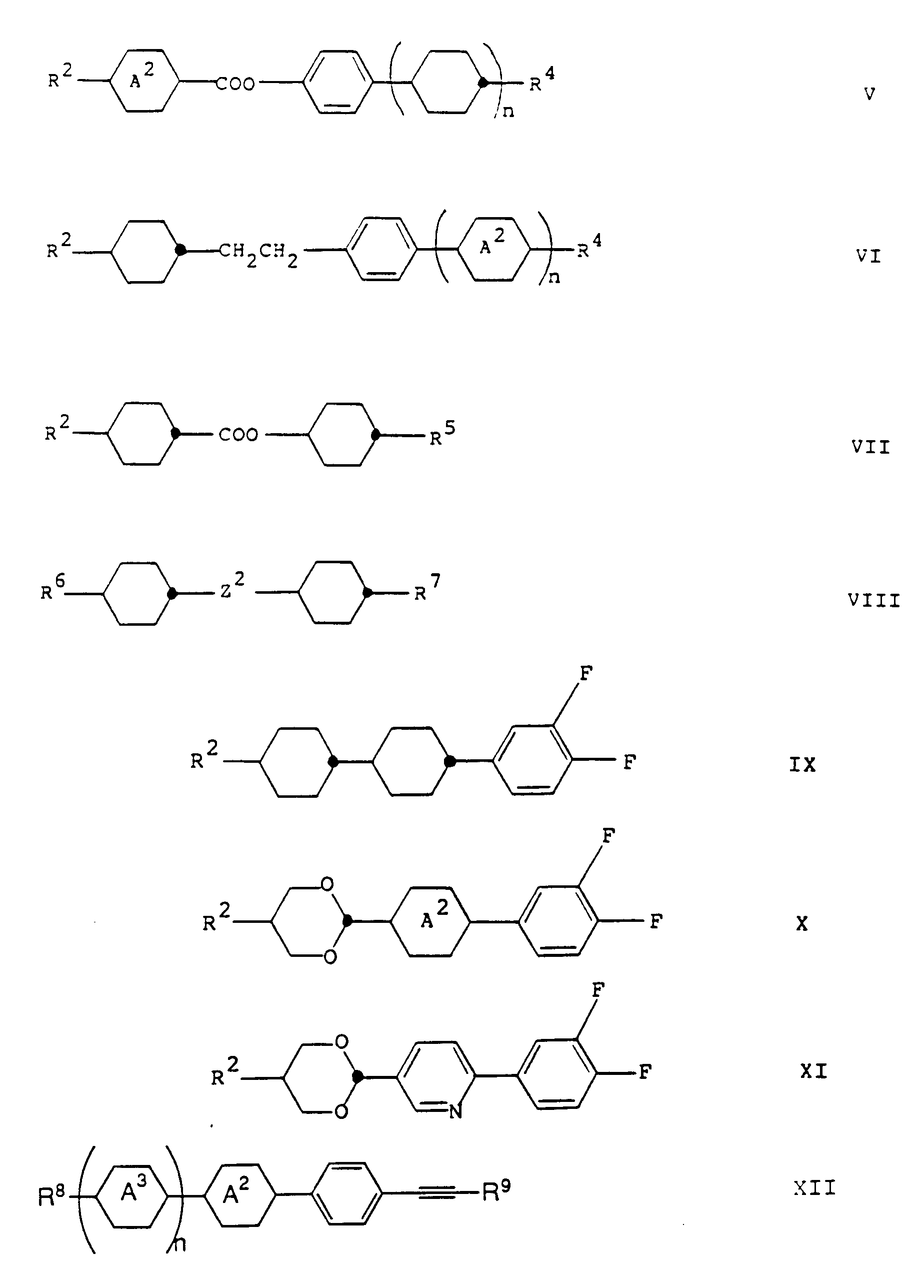 worin n für die Zahl 0 oder 1 steht; R² und R⁵ unabhängig voneinander Alkyl, 3E-Alkenyl, 4-Alkenyl oder an einem gesättigten Ring auch 1E-Alkenyl bedeutet; Ring A¹ 1,4-Phenylen, trans-1,4-Cyclohexylen, Pyrimidin-2,5-diyl oder trans-1,3-Dioxan-2,5-diyl darstellt; R³ Cyano, -NCS, Fluor, Alkyl, Difluormethoxy, Trifluormethoxy, 3E-Alkenyl, 4-Alkenyl, Alkoxy, 2E-Alkenyloxy oder 3-Alkenyloxy bezeichnet; Ring A² 1,4-Phenylen oder trans-1,4-Cyclohexylen darstellt; R⁴ Alkyl, Difluormethoxy, Trifluormethoxy, 3E-Alkenyl, 4-Alkenyl oder an einem Cyclohexanring auch 1E-Alkenyl oder an einem Benzolring auch Cyano, -NCS, Alkoxy, 2E-Alkenyloxy oder 3-Alkenyloxy bedeutet; R⁶ Alkyl, 1E-Alkenyl, 3E-Alkenyl oder 4-Alkenyl bezeichnet; Z² eine einfache Kovalenzbindung oder -CH₂CH₂ - darstellt; und R⁷ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl bedeutet; A³ trans-1,4-Cyclohexylen oder trans-1,3-dioxan-2,5-diyl darstellt; R⁸ 3E-Alkenyl, 4-Alkenyl oder, falls n für die Zahl 1 steht, auch 1E-Alkenyl bedeutet; und R⁹ Alkyl bedeutet;
enthält.

8. Verfahren zur Herstellung der Verbindungen der in Anspruch 1 definierten Formel I, dadurch gekennzeichnet, dass man einen Aldehyd der allgemeinen Formel 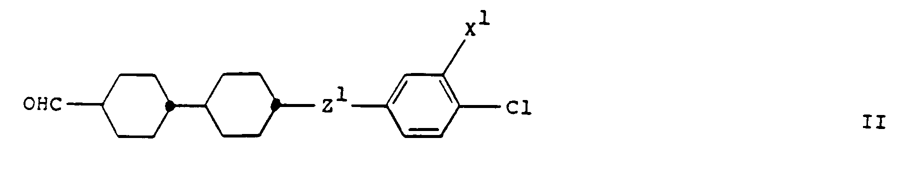 worin X¹ und Z¹ die in Anspruch 1 gegebenen Bedeutungen haben,
in Gegenwart einer Base mit Alkyltriphenylphosphoniumhalogenid umsetzt.

9. Verwendung der Verbindungen der in Anspruch 1 definierten Formel I für elektro-optische Zwecke.

## Claims

1. Compounds of the general formula 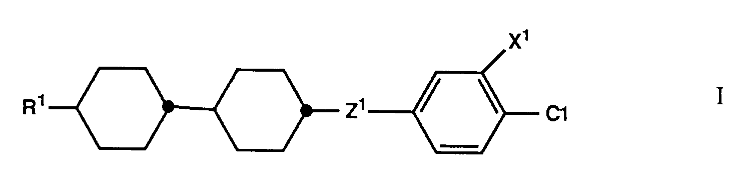 wherein Z¹ represents a single covalent bond or -CH₂CH₂-, X¹ denotes hydrogen, fluorine or chlorine and R¹ signifies 1E-alkenyl with 2 to 12 carbon atoms, with the exception of 1-[trans-4-(trans-4-vinylcyclohexyl)cyclohexyl]-4-chlorobenzene and 1-[trans-4-(trans-4-vinylcyclohexyl)cyclohexyl]-3-fluoro-4-chlorobenzene.

2. Compounds according to claim 1, characterized in that R¹ signifies a straight-chain residue.

3. Compounds according to claim 1 or 2, characterized in that R¹ has 2-7, preferably 2-5, carbon atoms.

4. A liquid crystalline mixture having at least 2 components, characterized in that at least one component is a compound of formula I defined in claim 1.

5. A liquid crystalline mixture according to claim 4, characterized in that the content of compounds of formula I amounts to 1-50 wt.%, preferably 5-30 wt.%.

6. A liquid crystalline mixture according to claim 4 or 5, characterized in that it contains one or more compounds of formula I and one or more compounds from the group of compounds of the general formulae 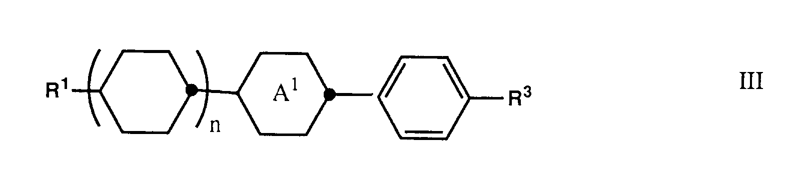 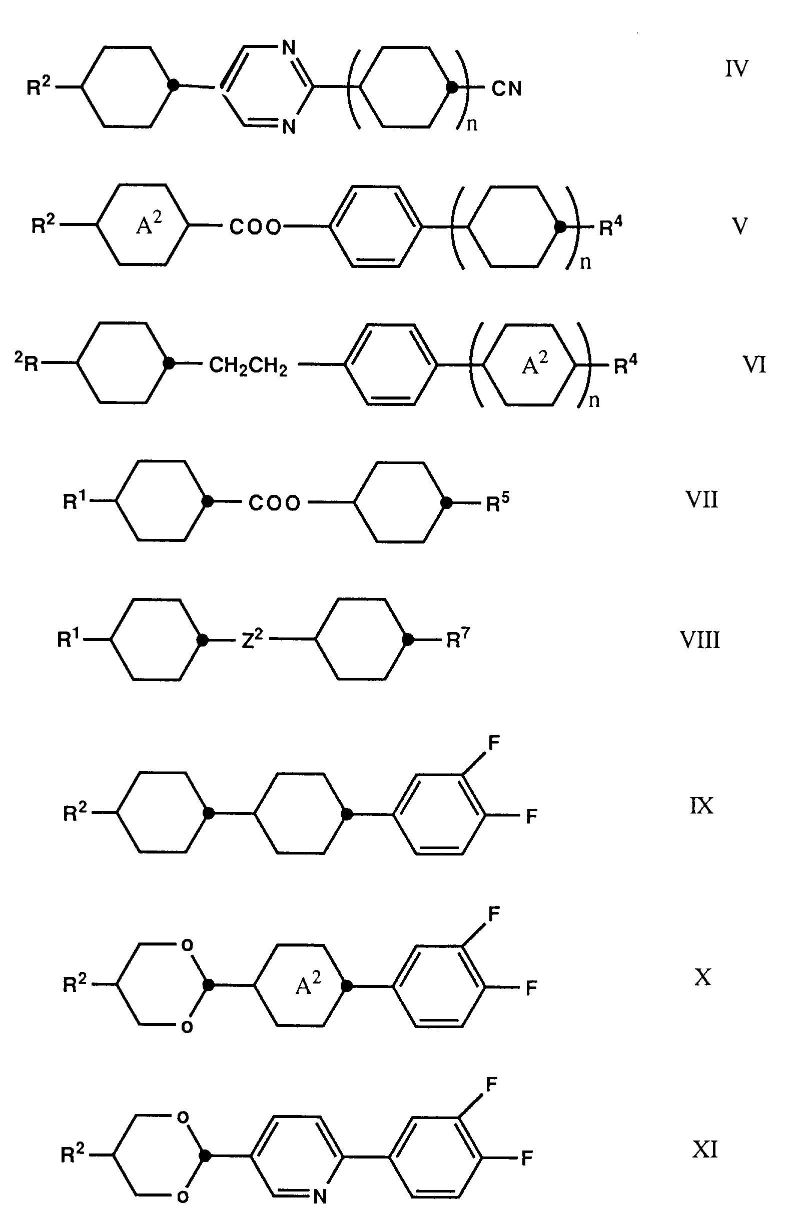 wherein n stands for the number 0 or 1; R² and R⁵ each individually signify alkyl, 3E-alkenyl, 4-alkenyl or on a saturated ring also 1E-alkenyl; ring A¹ represents 1,4-phenylene, trans-1,4-cyclohexylene, pyrimidine-2,5-diyl or trans-1,3-dioxane-2,5-diyl; R³ denotes cyano, -NCS, fluorine, alkyl, 3E-alkenyl, 4-alkenyl, alkoxy, 2E-alkenyloxy or 3-alkenyloxy; ring A² represents 1,4-phenylene or trans-1,4-cyclohexylene; R⁴ signifies alkyl, 3E-alkenyl, 4-alkenyl or on a cyclohexane ring also 1E-alkenyl or on a benzene ring also cyano, -NCS, alkoxy, 2E-alkenyloxy or 3-alkenyloxy; R⁶ denotes alkyl, 1E-alkenyl, 3E-alkenyl or 4-alkenyl; Z² represents a single covalent bond or -CH₂CH₂-; and R⁷ signifies cyano, alkyl, 1E-alkenyl, 3E-alkenyl, 4-alkenyl, alkoxy, 2E-alkenyloxy, 3-alkenyloxy, alkoxymethyl or (2E-alkenyl)oxymethyl.

7. A liquid crystalline mixture according to claim 4 or 5, characterized in that it contains one or more compounds of formula I and one or more compounds from the group of compounds of the general formulae 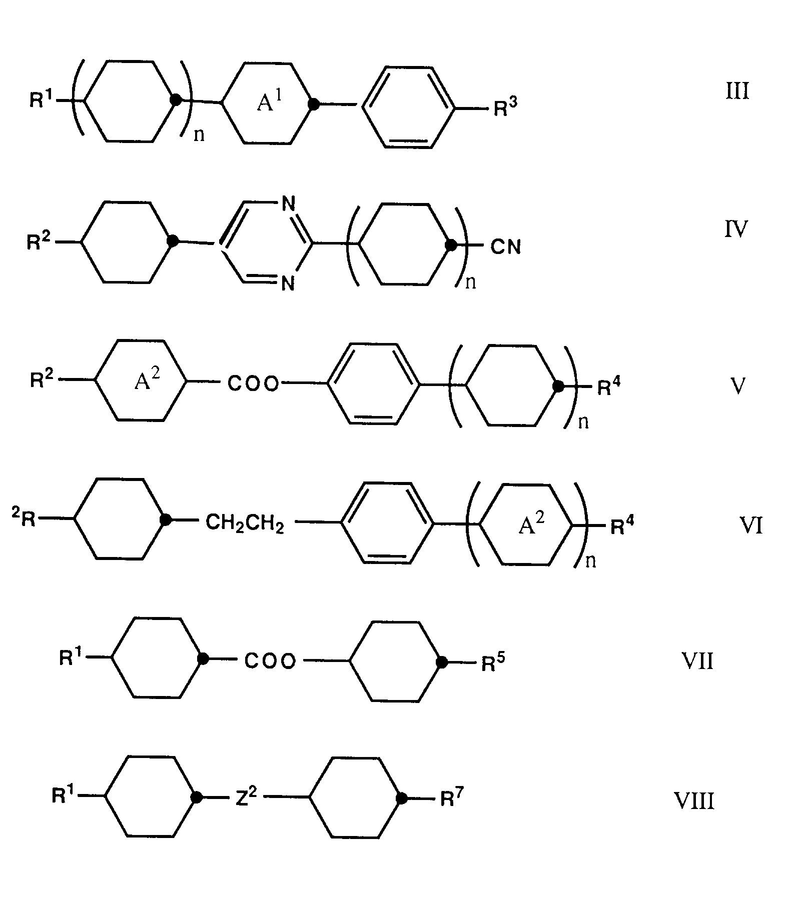 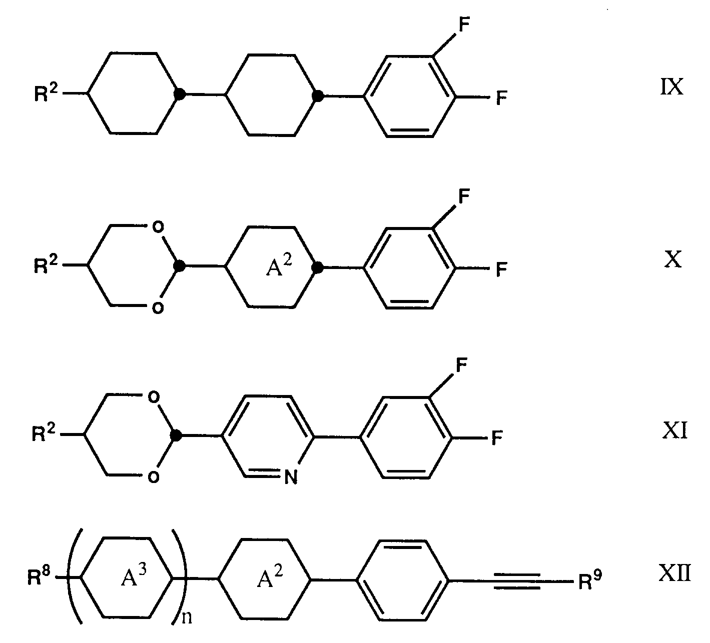 wherein n stands for the number 0 or 1; R² and R⁵ each individually signify alkyl, 3E-alkenyl, 4-alkenyl or on a saturated ring also 1E-alkenyl; ring A¹ represents 1,4-phenylene, trans-1,4-cyclohexylene, pyrimidine-2,5-diyl or trans-1,3-dioxane-2,5-diyl; R³ denotes cyano, -NCS, fluorine, alkyl, difluoromethoxy, trifluoromethoxy, 3E-alkenyl, 4-alkenyl, alkoxy, 2E-alkenyloxy or 3-alkenyloxy; ring A² represents 1,4-phenylene or trans-1,4-cyclohexylene; R⁴ signifies alkyl, difluoromethoxy, trifluoromethoxy, 3E-alkenyl, 4-alkenyl or on a cyclohexane ring also 1E-alkenyl or on a benzene ring also cyano, -NCS, alkoxy, 2E-alkenyloxy or 3-alkenyloxy; R⁶ denotes alkyl, 1E-alkenyl, 3E-alkenyl or 4-alkenyl; Z² represents a single covalent bond or -CH₂CH₂-; R⁷ signifies cyano, alkyl, 1E-alkenyl, 3E-alkenyl, 4-alkenyl, alkoxy, 2E-alkenyloxy, 3-alkenyloxy, alkoxymethyl or (2E-alkenyl)oxymethyl; A³ represents trans-1,4-cyclohexylene or trans-1,3-dioxane-2,5-diyl; R⁸ signifies 3E-alkenyl, 4-alkenyl or, where n stands for the number 1, also 1E-alkenyl; and R⁹ signifies alkyl.

8. A process for the manufacture of the compounds of formula I defined in claim 1, characterized by reacting an aldehyde of the general formula 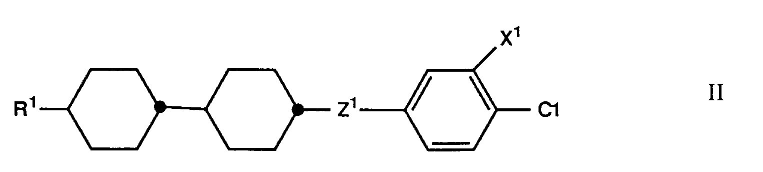 wherein X¹ and Z¹ have the significances given in claim 1, with alkyltriphenylphosphonium halide in the presence of a base.

9. The use of the compounds of formula I defined in claim 1 for electro-optical purposes.

## Revendications

1. Composés de formule générale 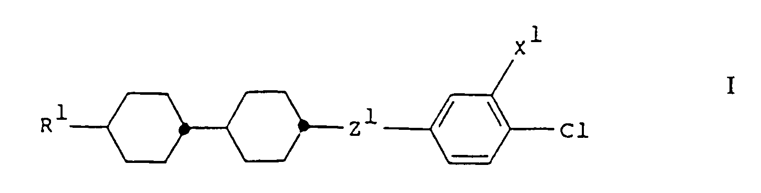 où Z¹ représente une liaison covalente simple ou -CH₂CH₂-, X¹ l'hydrogène, le fluor ou le chlore et R¹ un 1E-alcényle ayant 2 à 12 atomes de carbone, à l'exception du 1-[trans-4-(trans-4-vinylcyclohexyl)-cyclohexyl]-4-chlorobenzène et du 1-[trans-4-(trans-4-viynylcyclohexyl)-cyclohexyl]-3-fluoro-4-chlorobenzène.

2. Composés selon la revendication 1, caractérisés en ce que R¹ représente un reste à chaîne droite.

3. Composés selon la revendication 1 ou 2, caractérisés en ce que R¹ présente 2 à 7, de préférence, 2 à 5 atomes de carbone.

4. Mélange à cristaux liquides comportant au moins deux composants, caractérisé en ce qu'au moins un composant est un composé de formule I définie dans la revendication 1.

5. Mélange à cristaux liquides selon la revendication 4, caractérisé en ce qu'il contient 1 à 50 % en poids, de préférence, 5 à 30 % en poids de composés de formule I.

6. Mélange à cristaux liquides selon la revendication 4 ou 5, caractérisé en ce qu'il contient un ou plusieurs composés de formule I et un ou plusieurs composés provenant du groupe des composés de formules générales 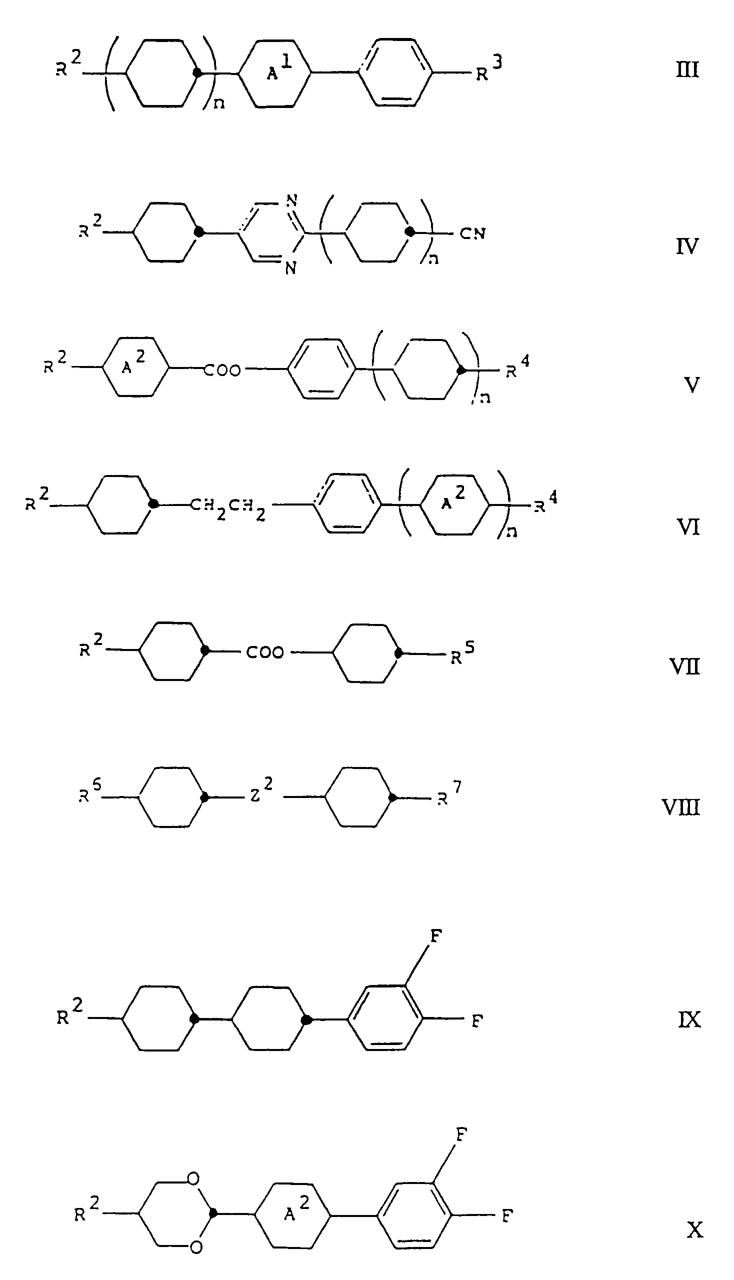 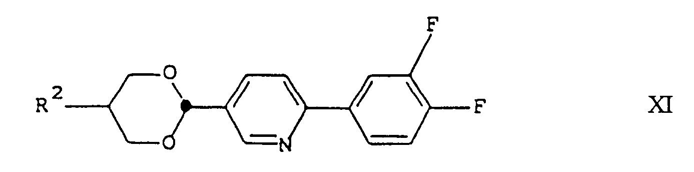 ou n est égal à 0 ou 1 ; R² et R⁵ représentent indépendamment l'un de l'autre un alkyle, un 3E-alcényle, un 4-alcényle ou sur un cycle saturé également un 1E-alcényle ; le noyau A¹ représente le 1,4-phénylène, le trans-1,4-cyclohexylène, le pyrimidine-2,5-diyle ou le trans-1,3-dioxane-2,5-diyle ; R³ représente le cyano, -NCS, le fluor, un alkyle, un 3E-alcényle, un 4-alcényle, un alcoxy, un 2E-alcényloxy ou un 3-alcényloxy ; le noyau A² représente le 1,4-phénylène ou le trans-1,4-cyclohexylène ; R⁴ représente un alkyle, un 3E-alcényle, un 4-alcényle ou sur un cycle cyclohexanique également un 1E-alcényle ou sur un noyau benzénique également le cyano, -NCS, un alcoxy, un 2E-alcényloxy ou un 3-alcényloxy ; R⁶ représente un alkyle, un 1E-alcényle, un 3E-alcényle ou un 4-alcényle ; Z² représente une liason covalente simple ou -CH₂CH₂- ; et R⁷ représente le cyano, un alkyle, un 1E-alcényle, un 3E-alcényle, un 4-alcényle, un alcoxy, un 2E-alcényloxy, un 3-alcényloxy, un alcoxyméthyle ou un (2E-alcényl)oxyméthyle.

7. Mélange à cristaux liquides selon la revendication 4 ou 5, caractérisé en ce qu'il contient un ou plusieurs composés de formule I et un ou plusieurs composés provenant du groupe de composés de formules générales 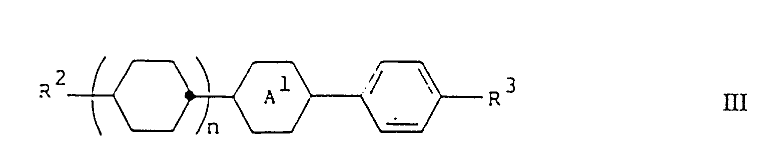 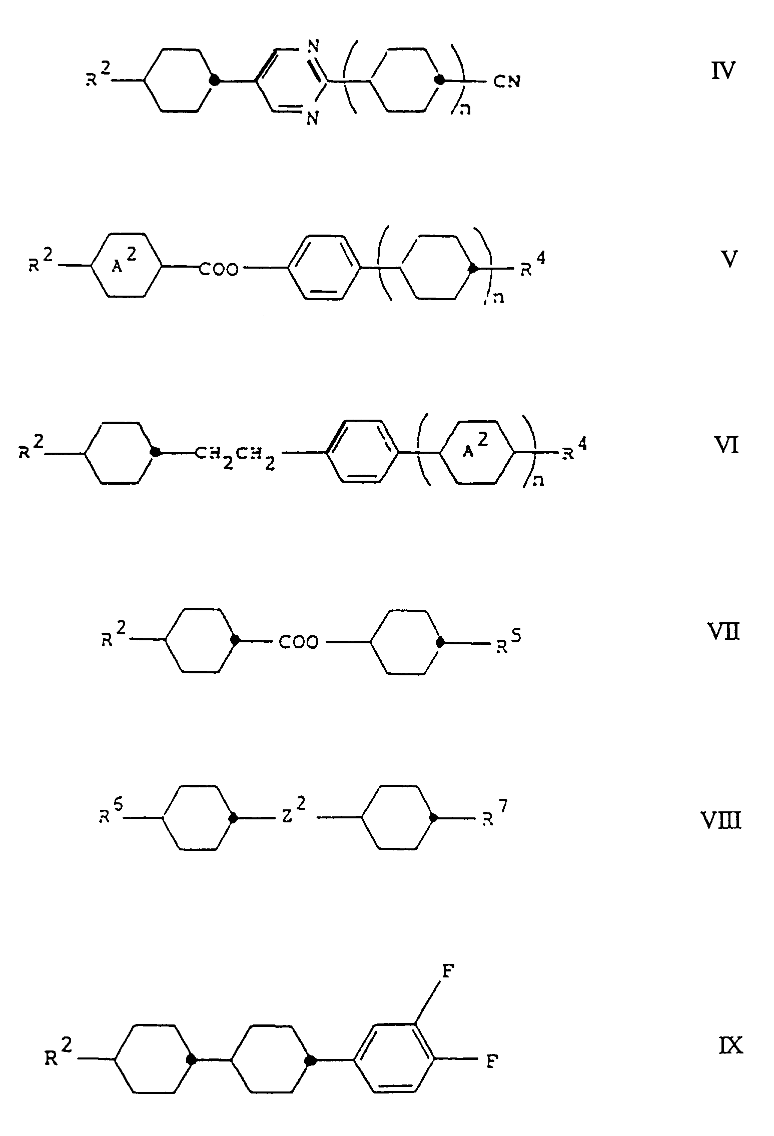 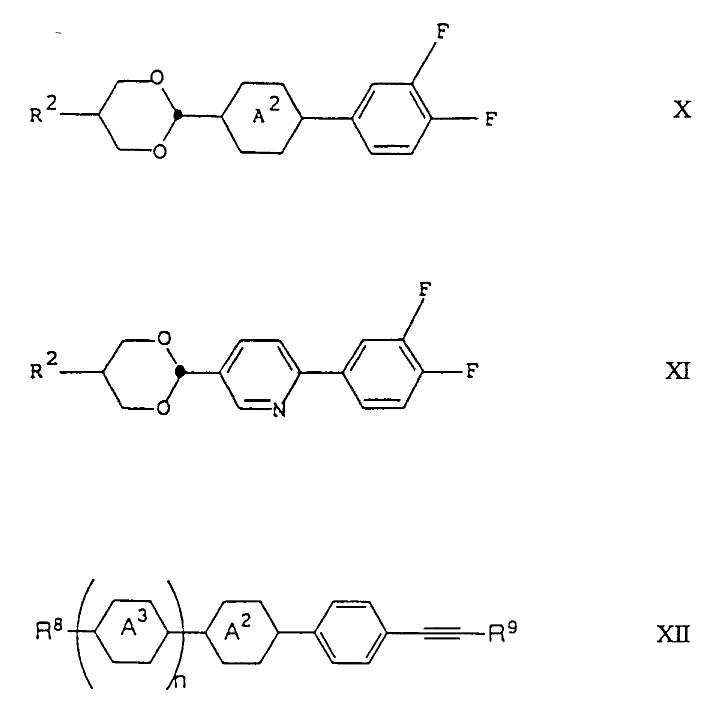 où n est égal à 0 ou 1; R² et R⁵ représentent indépendamment l'un de l'autre un alkyle, un 3E-alcényle, un 4-alcényle ou sur un cycle saturé également un 1E-alcényle ; le noyau A¹ représente le 1,4-phénylène, le trans-1,4-cyclohexylène, le pyrimidine-2,5-diyle ou le trans-1,3-dioxane-2,5-diyle ; R³ représente le cyano, -NCS, le fluor, un alkyle, le difluorométhoxy, le trifluorométhoxy, un 3E-alcényle, un 4-alcényle, un alcoxy, un 2E-alcényloxy ou un 3-alcényloxy ; le noyau A² représente le 1,4-phénylène ou le trans-1,4-cyclohexylène ; R⁴ représente un alkyle, le difluorométhoxy, le trifluorométhoxy, un 3E-alcényle, un 4-alcényle ou sur un cycle cyclohexanique également un 1E-alcényle ou sur un noyau benzénique également le cyano, -NCS, un alcoxy, un 2E-alcényloxy ou un 3-alcényloxy ; R⁶ représente un alkyle, un 1E-alcényle, un 3E-alcényle ou un 4-alcényle ; Z² représente une liaison covalente simple ou -CH₂CH₂- ; et R⁷ représente le cyano, un alkyle, un 1E-alcényle, un 3E-alcényle, un 4-alcényle, un alcoxy, un 2E-alcényloxy, un 3-alcényloxy, un alcoxyméthyle ou un (2E-alcényl)-oxyméthyle ; A³ représente le trans-1,4-cyclohexylène ou le trans-1,3-dioxane-2,5-diyle; R⁸ représente un 3E-alcényle, un 4-alcényle ou, si n est égal à 1, également un 1E-alcényle ; et R⁹ représente un alkyle.

8. Procédé pour la préparation des composés de formule I définie dans la revendication 1, caractérisé en ce que l'on fait réagir un aldéhyde de formule générale 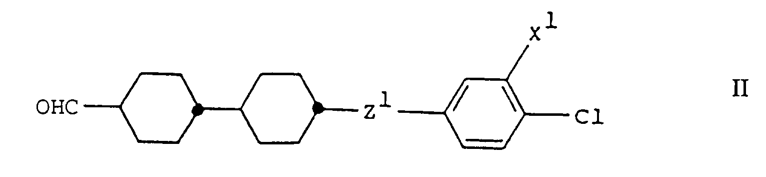 où X¹ et Z¹ ont les significations données dans la revendication 1, en présence d'une base avec un halogénure d'alkyltriphénylphosphonium.

9. Utilisation des composés de formule I définie dans la revendication 1 à des fins électro-optiques.
